(19) Europäisches Patentamt
European Patent Office
Office européen des brevets

(11) Publication number: **0 288 144 B1**

(12) **EUROPEAN PATENT SPECIFICATION**

(45) Date of publication of patent specification: **12.05.93**

(51) Int. Cl.[5]: **C07C 49/255**, C07C 45/65, C07C 45/62, C07C 45/72, C07C 45/74

(21) Application number: **88302285.7**

(22) Date of filing: **16.03.88**

The file contains technical information submitted after the application was filed and not included in this specification

(54) **3-(6'-Methoxy-2'-naphthylmethyl)-2,4 pentane dione, process for its preparation and intermediates.**

(30) Priority: **26.03.87 GB 8707200**

(43) Date of publication of application:
**26.10.88 Bulletin 88/43**

(45) Publication of the grant of the patent:
**12.05.93 Bulletin 93/19**

(84) Designated Contracting States:
**AT BE CH DE ES FR GB GR IT LI LU NL SE**

(56) References cited:
**AT-A- 343 643**
**DE-A- 2 035 334**
**GB-A- 1 504 442**
**US-A- 4 061 779**

**J. MARCH, "ADVANCED ORGANIC CHEMISTRY", 2nd edition, 1977, page 1116**

(73) Proprietor: **BEECHAM GROUP PLC**
**Beecham House Great West Road**
**Brentford Middlesex TW8 9BD(GB)**

(72) Inventor: **Callander, Sidney Edward**
**Beecham Pharmaceuticals Clarendon Road**
**Worthing West Sussex BN14 8OH(GB)**

(74) Representative: **Russell, Brian John et al**
**Beecham Pharmaceuticals Great Burgh Yew Tree Bottom Road**
**Epsom Surrey KT18 5XO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 288 144 B1

## Description

This invention relates to a novel process for preparing an intermediate compound useful for preparing 4-(6′-methoxy-2′-naphthyl)butan-2-one.

U.S. Patent 4061779 describes 4-(6′-methoxy-2′-naphthyl)butan-2-one (nabumetone) and its use in the treatment of rheumatic and arthritic conditions. A number of processes for preparing the compound are also described, one of which proceeds via a saturated diketone intermediate of formula (I), although there is no specific disclosure of this intermediate, nor of its structure. In the above patent, the compound of formula (I) is produced in a reaction medium comprising acetylacetone and potassium carbonate (Example 22 of the patent), but the compound is not disclosed as being isolated from this medium.

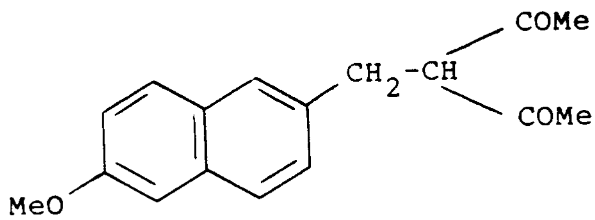

(I)

A further process for the preparation of the saturated diketone intermediate of formula (I) has now been discovered which is characterised by the reduction of a product formed by the condensation of 6-methoxy-2-naphthaldehyde with acetylacetone.

Accordingly, the present invention provides a process for the preparation of the compound of formula (I), 3-(6′-methoxy-2′-naphthylmethyl-2,4-pentanedione, which comprises the reduction of a compound of formula (II):

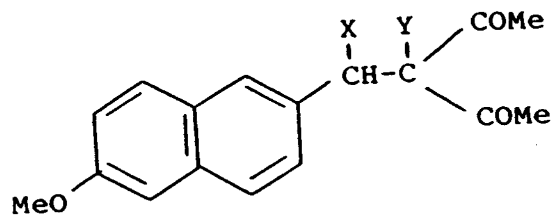

(II)

wherein X is a hydroxyl group and Y is hydrogen or X and Y together form a bond.

The compound of formula (I) thereby produced may then be de-acetylated under basic conditions to produce nabumetone.

The compound of formula (II) may itself be produced by the condensation of 6-methoxy-2-naphthaldehyde with acetylacetone, preferably in an inert solvent or solvent mixture. A suitable solvent is one which forms an azeotrope with water.

The condensation reaction may be carried out at a pressure of from $10^3$ to $10^6$ $Nm^{-2}$, and at a temperature of from $-5°$ to $100°C$. It is believed that the condensation reaction proceeds via the intermediate compound of formula (II) in which X is a hydroxyl group and Y is hydrogen, and that this intermediate spontaneously eliminates water to produce the $\alpha,\beta$-unsaturated diketone intermediate of formula (II) in which X and Y together form a bond.

In a preferred aspect, the condensation of 6-methoxy-2-naphthaldehyde with acetylacetone is carried out in toluene at a reduced pressure of about $7x10^3Nm^{-2}$ at the reflux temperature of the solvent, with azeotropic removal of water, to give the $\alpha,\beta$-unsaturated diketone intermediate compound of formula (II) which is isolated by cooling and filtration.

The reduction of the compound of formula (II) may be carried out by catalytic hydrogenation, employing a pressure, for example in the range $0.1x10^5$ to $10^6$ $Nm^{-2}$. In general it is preferred to use a hydrogen pressure in the range $0.9x10^5$ to $1.5x10^5$ $Nm^{-2}$, for example atmospheric pressure.

The hydrogenation will be carried out in the presence of a catalyst, preferably a noble metal catalyst such as palladium. Suitable forms of palladium catalysts include palladium on charcoal, palladium on barium sulphate or palladium on calcium carbonate. Palladium on charcoal is particularly preferred.

The hydrogenation reaction is preferably carried out in an inert solvent, such as a lower alcohol, weak acid, ester, hydrocarbon (such as toluene), halohydrocarbon or ketonic solvent, or a suitable mixture of solvents. The reaction may take place in an aqueous medium. Preferably the reaction is carried out in ethyl acetate.

The reaction may be performed at temperatures of, for example 20 ° C to 100 ° C and more suitably 45 ° C to 65 ° C, preferably 60 ° C.

Once the reaction is over (for example as judged by hplc, tlc or cessation of hydrogen uptake) the desired compound may be obtained by filtering off the catalyst and removal of the solvent, for example by evaporation under reduced pressure.

If desired the compound may be purified by recrystallisation or directly de-acetylated to produce nabumetone.

Compounds of formula (II) are novel and form an aspect of the present invention. The isolated compound of formula (I) is also novel and forms a further aspect of the present invention. The following examples illustrate the invention.

## Example 1

### 3-(6'Methoxy-2'-naphthylmethylene)-2,4-pentanedione

Piperidine (6.32 g), acetic acid (4.52 g), acetylacetone (118 g) and 6-methoxy-2-naphthaldehyde (200 g) were added to toluene (500 ml) in a glass reactor equipped with a stirrer and a Dean and Stark reflux system.

The pressure was reduced to ca $7 \times 10^3$ $Nm^{-2}$, and heating applied so that reflux started at ca 40 ° C. Water liberated from the reaction was removed continuously, and after ca 5 hours, the reaction mixture was cooled to 0-5 ° C. The crystalline product was isolated by filtration after a further 2 hours, and dried at 45 ° C.

| | |
|---|---|
| Yield: | 267.3 g |
| Assay: | 99.1% |
| Activity Yield: | 93.7% |
| Melting Point: | 121-2 ° C |

The structure of the product was confirmed by proton NMR, as follows:

| Line Position | Protons | Assignment |
|---|---|---|
| 2.35 ppm | 3 | $-COCH_3$ |
| 2.45 ppm | 3 | $-COCH_3$ |
| 3.9 ppm | 3 | $-OCH_3$ |

## Example 2

### 3-(6'-Methoxy-2'-naphthylmethyl)-2,4-pentanedione

3-(6'-Methoxy-2'-naphthylmethylene)-2,4-pentanedione (50 g) was added to ethyl acetate (250 ml) in a glass reactor fitted with a reflux condenser, a gas inlet tube, and stirrer.

The mixture was heated to ca 60 ° C while purging with nitrogen, and the catalyst (10% palladium on carbon, 0.8 g) was added as a slurry in ethyl acetate (25 ml).

The nitrogen flow was stopped and hydrogen was passed through the hot solution, with vigorous stirring, for ca 1 hour. The vessel was then purged with nitrogen while cooling the mixture to ca 30 ° C, and the catalyst was removed by filtration.

The ethyl acetate was distilled off under reduced pressure, and the oily residue was treated with isopropyl ether. The resulting white solid was isolated and dried at 45 °C. Yield 50 g.

Alternatively, the filtrate was concentrated, then cooled to allow crystallisation of the material. (M.p. 75–7 °C.).

The structure of the product was confirmed by proton NMR, as follows:

| Line Position | Protons | Assignment |
|---|---|---|
| 2.13 ppm | 6 | $-COCH_3$ |
| 3.9 ppm | 3 | $-OCH_3$ |

## Claims

**1.** A process for the preparation of 4-(6'-methoxy-2'-naphthyl)butan-2-one which comprises;

i) condensing 6-methoxy-2-naphthaldehyde with acetylacetone to form a compound of formula (II);

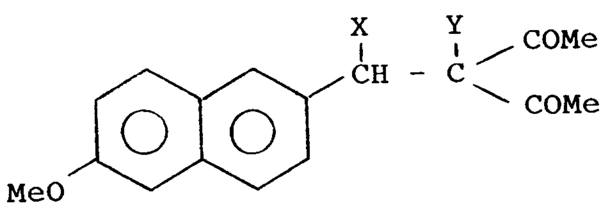

(II)

wherein X is a hydroxyl group and Y is hydrogen or X and Y together form a bond, followed by;

ii) the reduction of a compound of formula (II) to form a compound of formula (I);

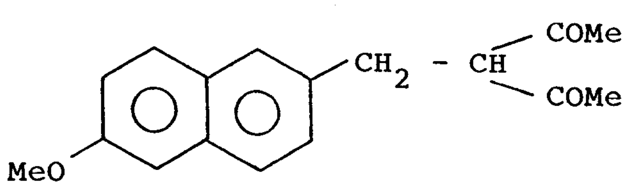

(I)

followed by;

iii) the deacetylation of a compound of formula (I) to form 4-(6'-methoxy-2'-naphthyl)butan-2-one.

**2.** A process according to claim 1, in which the condensation step i) is carried out at reduced pressure at reflux temperature of the solvent.

**3.** A process according to claims 1 or 2 in which the solvent utilised is toluene.

**4.** A process according to claim 1 in which the reduction step ii) is carried out by catalytic hydrogenation.

**5.** A process according to claim 4 in which the pressure of hydrogen is from $0.1 \times 10^5$ to $10^6$ $Nm^{-2}$.

6. A process according to any one of claims 1, 4 or 5 in which the catalyst comprises palladium.

7. A compound of formula (II):

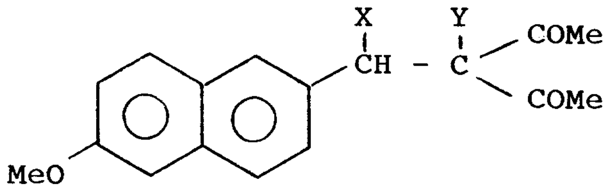

(II)

in which X is a hydroxyl group and Y is hydrogen or X and Y together form a bond.

8. Isolated 3 – (6' – methoxy – 2' – naphthylmethyl) – 2,4 – pentanedione.

**Patentansprüche**

1. Verfahren zur Herstellung von 4 – (6' – Methoxy – 2' – naphthyl)butan – 2 – on, umfassend
   i) die Kondensation von 6 – Methoxy – 2 – naphthaldehyd mit Acetylaceton unter Bildung einer Verbindung der Formel (II)

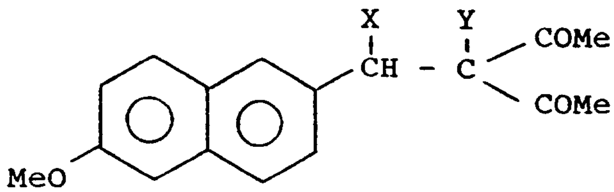

(II),

   in der X eine Hydroxylgruppe ist und Y ein Wasserstoffatom darstellt oder X und Y zusammen eine Bindung bilden; anschließend
   ii) die Reduktion der Verbindung der Formel (II) unter Bildung einer Verbindung der Formel (I)

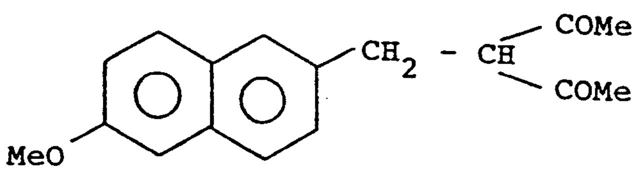

(I);

   anschließend
   iii) die Deacetylierung der Verbindung der Formel (I) unter Bildung von 4 – (6' – Methoxy – 2' – naphthyl)butan – 2 – on.

2. Verfahren gemäß Anspruch 1, in dem der Kondensationsschritt i) unter vermindertem Druck bei der Rückflußtemperatur des Lösungsmittels durchgeführt wird.

3. Verfahren gemäß den Ansprüchen 1 oder 2, in dem das verwendete Lösungsmittel Toluol ist.

4. Verfahren gemäß Anspruch 1, in dem der Reduktionsschritt ii) durch katalytische Hydrierung erfolgt.

5. Verfahren gemäß Anspruch 4, in dem der Wasserstoffdruck 0,1 x $10^5$ bis $10^6$ $Nm^{-2}$ beträgt.

6. Verfahren gemäß einem der Ansprüche 1, 4 oder 5, in dem der Katalysator Palladium umfaßt.

7. Eine Verbindung der Formel (II)

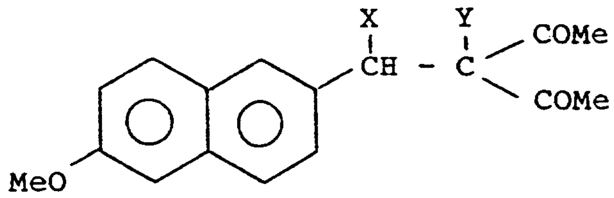

(II),

in der X eine Hydroxylgruppe ist und Y ein Wasserstoffatom bedeutet oder X und Y zusammen eine Bindung bilden.

8. Isoliertes 3-(6'-Methoxy-2'-naphthylmethyl)-2,4-pentandion.

**Revendications**

1. Procédé pour la préparation de la 4-(6'-méthoxy-2'-naphtyl)butan-2-one qui comprend :
(i) la condensation du 6-méthoxy-2-naphtylaldéhyde avec l'acétylacétone pour former un composé de formule (II) :

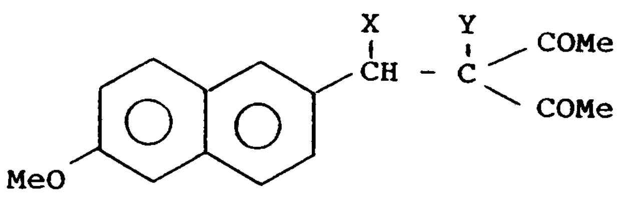

(II)

dans lequel X est un groupe hydroxy et Y est un atome d'hydrogène ou bien X et Y forment ensemble une liaison, puis
(ii) la réduction d'un composé de formule (II) pour former un composé de formule (I) :

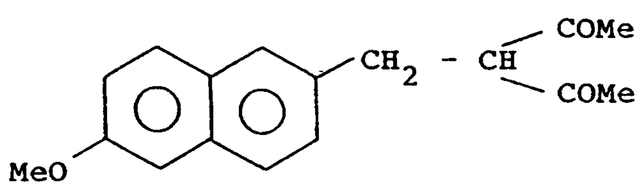

(I)

puis
(iii) la désacétylation du composé de formule (I) pour former la 4-(6'-méthoxy-2'-naphtyl)-butan-2-one.

2. Procédé suivant la revendication 1, dans lequel l'étape (i) de condensation est effectuée sous pression réduite à la température de reflux du solvant.

3. Procédé suivant les revendications 1 ou 2, dans lequel le solvant utilisé est le toluène.

**4.** Procédé suivant la revendication 1, dans lequel l'étape (ii) de réduction est effectuée par hydrogénation catalytique.

**5.** Procédé suivant la revendication 4, dans lequel la pression d'hydrogène est de 0,1 x $10^5$ à $10^6$ $Nm^{-2}$

**6.** Procédé suivant l'une quelconque des revendications 1, 4 ou 5, dans lequel le catalyseur comprend du palladium.

**7.** Composé de formule (II) :

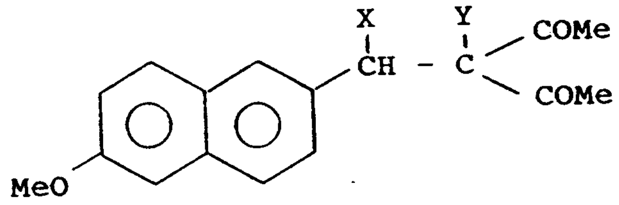

(II)

dans laquelle X est un groupe hydroxy et Y est un atome d'hydrogène ou bien X et Y forment ensemble une liaison.

**8.** 3 – (6' – méthoxy – 2' – naphtylméthyl) – 2,4 – pentanedione isolée.